# FASCICULE DE BREVET EUROPEEN

(11) **EP 1 814 523 B1**
(45) Date de publication et mention de la délivrance du brevet: **25.08.2010**
(21) Numéro de dépôt: 05819409.3
(22) Date de dépôt: 21.11.2005
(51) Int. Cl.: A61K 9/16, A61K 9/20, A61K 47/44, A61K 47/32, A61K 47/36

(54) **FORME MEDICAMENTEUSE ORALE, SOLIDE ET CONCUE POUR EVITER LE MESUSAGE**
ZUR VERHINDERUNG VON MISSBRAUCH ENTWORFENE, FESTE ARZNEIMITTELFORM ZUR ORALEN VERABREICHUNG
SOLID, ORAL DRUG FORM WHICH HAS BEEN DESIGNED TO PREVENT MISUSE

(30) Priorité: 23.11.2004 FR 0412428
(43) Date de publication de la demande: 08.08.2007
(73) Titulaire: FLAMEL TECHNOLOGIES, 69200 Vénissieux (FR)
(72) Inventeur: SOULA, Gérard, F-69330 MEYZIEU (FR); DARGELAS, Frédéric, F-33600 PESSAC (FR)
(74) Mandataire: Fleurance, Raphaël
(86) Numéro de dépôt international: PCT/FR2005/050969
(87) Numéro de publication internationale: WO 2006/056712

(56) Documents cités:
- EP-A- 0 198 769
- WO-A-2005/016313
- WO-A-2005/016314
- US-A- 4 070 494
- US-B1- 6 309 668

## Description

### Domaine de l'invention

Le domaine de la présente invention est celui des médicaments solides destinés à une administration par voie orale de principes actifs.

Les principes actifs considérés (PA) sont des PA pharmaceutiques, et notamment ceux classés dans la catégorie des produits stupéfiants. Ces derniers sont ceux dont l'abus peut donner lieu à des conduites toxicomaniaques.

Au sens du présent exposé, l'expression "PA", désigne aussi bien un seul principe actif, qu'un mélange de plusieurs principes actifs.

Le but visé par la présente invention est de prévenir le détournement des médicaments solides oraux, pour tout autre usage que l'usage ou les usages thérapeutiques officiellement approuvés par les autorités de santé publique compétentes. En d'autres termes, il s'agit d'éviter le mésusage volontaire ou involontaire des médicaments solides oraux.

Position du Problème Le mésusage se rencontre principalement dans les cas suivants:
a. comportement addictif (toxicomanie, dopage),
b. comportement criminel (asservissement chimique).
c. utilisation d'un médicament de façon non conforme aux recommandations médicales (posologie), par mégarde ou du fait d'invalidités affectant le patient,
d. automédication.

Dans le cas a. (voire b.), les personnes ayant l'intention de faire un mésusage du médicament solide, oral, vont généralement s'employer à le mettre soit sous une forme pulvérulente pouvant être inhalée, soit sous une forme liquide injectable à l'aide d'une seringue.

La transformation solide/poudre à priser s'effectue par broyage. L'obtention d'une forme liquide injectable à partir d'un médicament oral solide, passe par une étape d'extraction aqueuse ou alcoolique du PA visé. Cette extraction peut être précédée d'un broyage.

Les modes d'administration par inhalation ou par injection, conviennent particulièrement bien aux toxicomanes car ce sont des modes qui permettent d'accentuer les effets du PA et qui favorisent son absorption dans l'organisme sur des temps courts. Lorsque cette poudre est aspirée par le nez ou dissoute dans de l'eau et injectée, les effets recherchés, dopants ou euphorisants, du PA, se manifestent très rapidement et de manière exacerbée.

Le mésusage de médicaments oraux solides peut également être observé lorsque le médicament est mastiqué avant d'être avalé, au lieu d'être avalé rapidement conformément à la posologie.Cela est particulièrement observé dans le cas des formes médicamenteuses à libération prolongée où la dose de principe actif par présentation galénique (comprimé, gélule) peut être relativement élevée par rapport à des produits classiques à libération immédiate. En effet, un médicament à libération prolongée de PA contient une dose supérieure de PA car il doit couvrir les besoins sur une période plus longue (ex : administration d'une même quantité de principe actif en une seule prise par jour au lieu de plusieurs). Le fait de croquer ou de broyer le médicament et de l'avaler ne garantit plus la libération contrôlée initiale et permet ainsi une administration massive de PA provoquant les effets dopants ou euphorisants recherchés.

Les risques liés aux comportements addictifs (a.) et criminels (b.) et à l'automédication (d.) sont évidents. On rappellera que le mésusage de médicaments par injection est aggravant: les excipients peuvent être responsables de nécroses locales des tissus, d'infections, de troubles respiratoires et cardiaques.

S'agissant des déviations (c.) de l'usage d'un médicament liées à l'inattention et/ou à des invalidités du patient, elles peuvent aussi avoir des conséquences sérieuses. Par exemple, la mastication avant déglutition de formes à libération modifiée de PA, transforme le médicament en une forme à libération immédiate. Ainsi, au mieux le médicament est inefficace après un temps très court et au pire il devient toxique.

Il existe donc clairement un grave problème de santé publique lié au mésusage des médicaments, et en particulier des médicaments oraux solides.

Ce phénomène en croissance inquiète de plus en plus les autorités sanitaires qui multiplient les appels au développement de formes médicamenteuses permettant la prévention du détournement.

### Art antérieur

A la connaissance de la demanderesse, les seules tentatives de réponse à ce problème ont consisté à adjoindre aux médicaments concernés des composés chimiquement actifs contre le mésusage.

Cette solution présente des dangers certains pour les utilisateurs, y compris pour un emploi dans les conditions approuvées. De surcroît, les combinaisons de PA et d'autres composés actifs sont délicates à maîtriser et sont accueillies avec méfiance par les autorités de santé publique chargées de délivrer les autorisations de mise sur le marché.

US-A-2003/0068371 décrit une formulation pharmaceutique orale comprenant un PA opiacé, un antagoniste de ce PA et un agent gélifiant (e.g. gomme xanthane). L'agent gélifiant est présenté comme conférant à la formulation une viscosité telle qu'elle ne puisse pas être administrable par voie nasale et parentérale. La présence de cet antagoniste est un inconvénient majeur, au regard des risques médicaux éventuellement encourus par les utilisateurs. En outre, cette forme pharmaceutique peut être mise sous forme pulvérulente et, par conséquent, peut être l'objet d'un mésusage par voie nasale.

EP-A-0 198 769 décrit une tablette pour la libération prolongée d'un principe actif capable de flotter dans le liquide gastrique grâce à sa densité, inférieure à celle de l'eau. Ces tablettes comprennent un principe actif thérapeutique, un agent gélifiant hydrocolloïdal et une huile inerte thérapeutiquement acceptable.

US-A-4 070 494 décrit des compositions pharmaceutiques entériques qui contiennent un principe actif susceptible d'être utilisé de façon abusive par voie parentérale. Ces compositions pharmaceutiques sont rendues résistantes au mésusage par extraction aqueuse grâce à l'incorporation d'une quantité suffisante d'un composant gélifiable dans l'eau. Des tentatives d'extraction du principe actif sont ainsi empêchées puisque le composant gélifiable forme un gel en présence d'eau et ne laisse pas de liquide filtrable.

D3: US-B-6 309 668 (BASTIN et al.) décrit des comprimés anti-mésusage contenant deux ou plusieurs couches à base d'un ou plusieurs principes actifs et d'un ou plusieurs agents gélifiants, et la préparation d'un tel comprimé multicouches. Les principes actifs et les agents gélifiants se trouvent dans des couches séparées du comprimé.

### Objectifs de l'invention

Dans ces circonstances, l'un des objectifs essentiels de la présente invention est de combler les lacunes de l'art antérieur.

Un autre objectif essentiel de l'invention est de fournir de nouveaux médicaments solides oraux, dont le mésusage sera rendu très difficile voire impossible, notamment pour les cas (a.)(b.)(c.)(d.) susévoqués, et ce sans recourir à des substances, autres que le PA. pouvant être pharmaceutiquement actives et donc dangereuses pour les utilisateurs.

Un autre objectif essentiel de l'invention est de fournir un nouveau médicament solide oral, permettant d'éviter le détournement frauduleux des propriétés du PA qu'il contient, en empêchant toute transformation du médicament donnant accès à des prises par les voies orales, nasales et/ou injectables (intra-veineuse, sous-cutanée, intra-musculaire....) hors du cadre thérapeutique. Ce faisant les risques associés à ces dérives seraient prévenus ou à tout le moins fortement réduits.

Un autre objectif essentiel de l'invention est de fournir un nouveau médicament solide oral, permettant d'éviter le mésusage tout en garantissant, pour le patient normalement suivi, une qualité de traitement, en particulier une close, conforme à ses besoins.

Un autre objectif essentiel de l'invention est de fournir un nouveau médicament solide oral, permettant d'éviter le mésusage, sans affecter les propriétés pharmacologiques du médicament, et sans faire courir de risques supplémentaires au patient utilisant normalement le médicament et enfin sans nuire au confort de ce dernier lors de l'administration.

Un autre objectif essentiel de l'invention est de fournir un nouveau médicament solide oral, permettant d'éviter le mésusage, qui soit simple a obtenir et qui ne grève pas son coût de revient.

### Description succincte de l'invention

Pour atteindre ces objectifs, les inventeurs ont eu le mérite de reformuler le problème à résoudre, en le posant non plus en termes chimiques, mais plutôt en termes physico-chimiques. Cette nouvelle approche leur a permis de découvrir, de manière surprenante et inattendue, qu'il convient de faire intervenir, dans le médicament dont on cherche à empêcher le mésusage, une combinaison d'agents dont le mode d'action est physico-chimique et qui ont vocation à contrarier, voire à rendre impossible, tout acte volontaire ou non de mésusage.

C'est ainsi que l'invention concerne, à titre principal, une forme médicamenteuse orale et solide, caractérisée en ce qu'elle comprend :
au moins un principe actif (PA) pharmaceutique, tout ou partie du principe actif de cette forme médicamenteuse étant contenu dans des microparticules,
des billes insolubles en milieu aqueux ou hydroalcoolique, incompressibles et inertes, de diamètre moyen supérieur ou égal à 1,25 fois, de préférence 1,5 fois, et plus préférentiellement encore 2 fois, le diamètre moyen des microparticules de principe actif,
   ainsi que
   A) au moins un agent mottant,
      et
   B) au moins un agent viscosifiant; de manière à éviter le mésusage.

La forme médicamenteuse selon l'invention résout le problème posé et satisfait aux objectifs fixés, de façon efficace, simple et économique, à l'aide de moyens physico-chimiques. Ces derniers sont totalement inoffensifs pour l'utilisateur normal. Ce sont des composés pharmacologiquement neutres (inertes), approuvés par la pharmacopée et par les autorités de santé publique chargées de délivrer les autorisations de mise sur le marché des médicaments.

L'agent A) mottant rend difficile le broyage de la forme médicamenteuse solide et massive

et ne permet pas d'obtenir une forme pulvérulente propre à l'administration par aspiration nasale.

L'agent B) viscosifiant rend le PA difficilement extractible de la forme médicamenteuse, prévenant ainsi le mésusage. Par ailleurs. B) rend difficile voire impossible son injection par voie parentérale.

### Description détaillée de l'invention

La présente invention concerne toutes les formes médicamenteuses orales, solides, unitaires ou divisées, à libération immédiate ou prolongée, empêchant le mésusage du médicament, en particulier du PA qu'il contient, aussi bien par injection (parentérale) que par voie nasale ou orale.

Ces formes peuvent être, par exemple, des comprimés ou des gélules.

En complément de la combinaison des agents A) et B), l'invention est caractérisée par les deux autres caractéristiques essentielles, ci-dessous détaillées.

Comme autre obstacle au mésusage, en complément des agents A) et B), la forme médicamenteuse selon l'invention comprend des billes insolubles, inertes, de diamètre moyen supérieur ou égal à 1,25 fois, de préférence 1,5 fois, et plus préférentiellement encore 2 fois, le diamètre moyen des microparticules de PA.

Ces billes insolubles, c'est à dire insolubles en milieu aqueux ou hydroalcoolique au sens de l'invention, sont incompressibles. Elles vont donc, du fait de leur taille plus importante que celle des microparticles renfermant le PA, supporter principalement les contraintes du broyage. Ainsi, les microparticules renfermant le PA seront préservées du broyage.

Suivant une caractéristique préférée de l'invention, l'agent mottant A) est choisi parmi ceux aptes à faire en sorte, en cas de broyage de la forme médicamenteuse, que celle-ci se transforme en produit non-pulvérulent.

En effet, comme indiqué ci-avant, le mésusage par inhalation nasale suppose que l'utilisateur broie la forme orale solide pour la transformer en une poudre à priser.

En outre, un auteur de mésusage peut également chercher à extraire le PA par voie aqueuse et/ou alcoolique, pour le concentrer.

Partant de ces constats, les inventeurs ont fait ce raisonnement inventif selon lequel il convenait de compliquer (voire d'empêcher) ces opérations de broyage (ou tout autre traitement mécanique permettant de transformer un solide en poudre) et d'extraction en mettant en oeuvre au moins un agent mottant ayant pour fonction de transformer la forme médicamenteuse en produit non pulvérulent, par exemple en pâte visqueuse non manipulable, dès qu'on lui fait quitter son état solide massif.

La forme médicamenteuse contient ainsi au moins un agent A) hydrophobe (cire, huile). Si le médicament détourné est broyé (pour être prisé en poudre), le composé hydrophobe joue le rôle d'un liant à sec. Le PA et les divers excipients forment un mélange qui ne peut pas être finement divisé (poudre lourde pâteuse) et qui empêche son aspiration par voie nasale.

De même, il est bien connu que l'extraction d'un composé à concentrer d'une pâte visqueuse est extrêmement difficile.

De manière plus préférée encore, l'agent mottant A) est choisi dans la classe des composés hydrophobes agissant comme agent liant à sec, de préférence:
→ dans le groupe comprenant les huiles de coton, les huiles de soja, les huiles de palme, les huiles de ricin et les mélanges de tout ou partie de ces huiles; et/ou
→ dans le groupe des cires, et plus préférentiellement encore dans le sous-groupe des cires comprenant les huiles de coton hydrogénées, les huiles de soja hydrogénées, les huiles de palme hydrogénées, les béhénates de glycérol, les huiles de ricin hydrogénées, les tristéarines, les tripalmitines, les trimyristines, les cires jaunes, les graisses dures, les matières grasses laitières anhydres, les lanolines, les palmitostéarates de glycérol, les stéarates de glycérol, les macrogolglycérides d'acide laurique, les alcools cétyliques, les diisostéarates de polyglycéryle, les monostéarates de diéthylène glycol, les monostéarates d'éthylène glycol, les omégas 3 et les mélanges de tout ou partie de ces cires; et/ou
→ dans le groupe des bases grasses pour suppositoires comprenant la glycérine, les triglycérides, les huiles de théobroma, les beurres de cacao et les mélanges de tout ou partie de ces produits.

S'agissant de la quantité d'agent mottant A) susceptible d'être introduite dans la forme médicamenteuse selon l'invention, on prévoit par exemple une concentration allant de 1 à 90% poids/poids par rapport à la masse totale de la forme médicamenteuse.

C'est donc une caractéristique remarquable de la forme médicamenteuse selon l'invention, que de ne pas être transformable en une forme sèche administrable par aspiration nasale.

De préférence, l'agent viscosifiant B) est choisi parmi ceux aptes à rendre non injectable(s), le(s) PA contenu(s) dans la forme médicamenteuse.

La mise en oeuvre de cet agent viscosifiant B) est plus spécialement (mais non limitativement) liée à un mésusage par injection parentérale du PA et des excipients d'une forme médicamenteuse.

En effet, l'auteur d'un tel mésusage doit transformer un produit solide en liquide injectable le plus concentré possible en PA stupéfiant. Comme expliqué supra, cela passe par une extraction aqueuse et/ou alcoolique. Le mésusage se poursuit par un remplissage d'une seringue avec le liquide obtenu, avant l'injection.

Dans ce contexte, l'idée originale des inventeurs a été de prévoir un agent B) viscosifiant, qui provoque, dès lors qu'il est mis en contact avec un liquide, une augmentation de la viscosité rendant impossible l'injection à l'aide d'une seringue. Cette forte viscosité empêche aussi bien le remplissage que la vidange de la seringue.

La forme médicamenteuse de l'invention contient au moins un agent B) avantageusement choisi parmi les polymères viscosifiants. Lorsque la forme médicamenteuse est mélangée avec un solvant (aqueux ou organique) le polymère B) participe à l'augmentation de la viscosité et/ou à la gélification du milieu limitant, d'une part, la dissolution du PA et empêchant, d'autre part, de prélever ou d'injecter la solution au moyen d'une seringue.

Il apparaît que, s'agissant de l'agent B) viscosifiant (à l'instar de l'agent mottant A)), le mécanisme faisant obstacle au mésusage par injection est purement physico-chimique et par conséquent neutre pour l'utilisateur normal du médicament.

De préférence, l'agent B) viscosifiant est choisi dans les groupes de polymères suivants :
→ les polyacides acryliques, et/ou
→ les polyalkylènes glycols (e.g. polyéthylène glycol), et/ou
→ les polyvinylpyrrolidones, et/ou
→ les gélatines, et/ou
→ les polysaccharides, de préférence dans le sous-groupe comprenant: l'alginate de sodium, les pectines, les guars, les xanthanes, les carraghénanes, les gellanes et les dérivés de la cellulose (e.g. hydroxypropylméthylcellulose, méthylcellulose, hydroxyéthylcellulose, carboxyméthylcellulose)
   et leurs mélanges.

S'agissant de la quantité d'agent B) viscosifiant susceptible d'être introduite dans la forme médicamenteuse selon l'invention, on prévoit par exemple une concentration allant de 1 à 90% poids/poids par rapport à la masse totale de la forme médicamenteuse.

C'est donc une caractéristique remarquable de la forme médicamenteuse selon l'invention, que de ne pas être transformable en une forme injectable.

Avantageusement, la forme médicamenteuse combine les agents A) et B) décrits ci-dessus pour prévenir le mésusage de façon adaptée à tout médicament et/ou à tout PA.

En résumé, l'emploi de A) et de B) dans la forme médicamenteuse selon l'invention la rend non prisable et non injectable.

Il n'est pas possible de la transformer ni en poudre volatile, ni en liquide concentré en PA pompable et expulsable à l'aide d'une seringue.

La nature multimicroparticulaire de la forme médicamenteuse et/ou la présence de billes insolubles inertes, contribue au résultat susvisé.

La forme médicamenteuse selon l'invention peut comprendre du PA à libération immédiate et/ou du PA à libération modifiée.

De préférence, les billes insolubles neutres sont choisies dans le groupe de matériaux suivants: les celluloses et leurs dérivés insolubles, les résines polyméthacryliques et leurs dérivés, les silices, le talc, la semoule de blé, la bentonite et leurs mélanges.

Comme précise ci-avant, les formes à libération modifiée de PA sont plus fortement dosées en PA que les formes à libération immédiate. Cela a généré, notamment de la part des toxicomanes, un mésusage consistant à broyer et/ou à mastiquer les formes à libération modifiée de PA, de façon à détruire les barrières prévues pour assurer la libération modifiée du PA et ainsi avoir accès à de plus fortes concentrations en PA stupéfiant.

Il convient de distinguer, d'une part, la mastication et, d'autre part, le broyage.

Concernant la mastication, il y a lieu de souligner qu'elle peut être volontaire ou involontaire. En effet, certains patients souffrant d'invalidités -e.g. maladie de parkinson- ne sont pas aptes à respecter la posologie qui prescrit d'avaler sans croquer.

Pour remédier à cela, l'invention propose une forme multimicroparticulaire, dans laquelle les microparticles de PA ont un diamètre moyen réduit, pour échapper à la mastication.

Concernant le broyage, il constitue une étape nécessaire et cruciale lors du détournement du médicament, quel que soit le mésusage, en préalable à l'extraction du PA.

Or, les caractéristiques propres à l'invention, à savoir combinaison A) et B), PA sous forme multimicroparticulaire et présence de billes insolubles inertes de plus grand diamètre que les microparticules de PA, protègent ces dernières de toute forme efficace de broyage. Il n'est donc pas possible de libérer le PA hors des microcapsules ni de l'extraire.

Ainsi, la présente invention propose un premier mode particulier de mise en oeuvre permettant de lutter efficacement contre le mésusage par mastication évoqué dans les paragraphes précédents.

Conformément à ce premier mode particulier de mise en oeuvre, tout ou partie du PA de la forme médicamenteuse selon l'invention est contenu dans des microparticules.

La présente invention propose également un second mode particulier de mise en oeuvre permettant de lutter efficacement contre le mésusage par broyage, évoqué dans les paragraphes précédents.

Conformément à ce second mode particulier de mise en oeuvre,
→ tout ou partie du PA de la forme médicamenteuse selon l'invention est contenu dans des microparticules,
→ la forme médicamenteuse comprend au moins un agent mottant A) et au moins un agent viscosifiant B),
→ la forme médicamenteuse comprend des billes insolubles et inertes, telles que définies ci-dessus.

Cette forme "multimicroparticulaire" présente l'avantage de fournir une dose de PA dispersée dans une pluralité de microparticules, de sorte que l'accès au PA privé de ses barrières de libération modifiée, par broyage et/ou mastication, est significativement restreint. En effet, un certain nombre des microparticules échappent à la destruction des moyens de libération modifiée du PA, du fait de leur très petite taille.

Dans le cas d'un comprimé matriciel dragéifié, il suffit de croquer le comprimé pour mettre à jour le coeur contenant le PA puis de l'avaler. Dans le cas d'une forme divisée composée de nombreuses microparticules, il faut fracturer individuellement l'enrobage d'un grand nombre d'objets sphériques dont la taille (de l'ordre de grandeur des interstices dentaires) est telle qu'elle est d'une part difficile à croquer et que d'autre part le phénomène naturel de salivation et de déglutition fait que les microparticules ont naturellement tendance à ne pas rester en bouche et à échapper à la mastication. Ce phénomène naturel peut être avantageusement amplifié par l'adjonction d'excipients comme les sucres, les acidifiants et agents de sapidité par exemple.

Une caractéristique remarquable de la forme médicamenteuse selon l'invention est que l'extraction du PA par mastication et/ou broyage n'est pas efficace.

Encore une fois, cette dispersité microparticulaire préconisée par l'invention est de nature physico-chimique et ne peut donc pas avoir d'effets néfastes à l'encontre des utilisateurs orthodoxes.

Suivant une disposition remarquable de l'invention, au moins une partie des microparticules de la forme médicamenteuse sont des microcapsules à libération modifiée de PA.

Ces microcapsules sont avantageusement constituées chacune d'un coeur comprenant du PA et d'un enrobage mono ou multicouche enveloppant le coeur et réagissant la libération modifiée du PA.

De préférence, la forme médicamenteuse "multimicroparticulaire" selon l'invention est caractérisée en ce que les microparticles et/ou les microcapsules ont un diamètre moyen inférieur ou égal à 1000 µm, de préférence inférieur ou égal à 500 µm et plus préférentiellement de diamètre inférieur ou égal à 300 µm.

Le mésusage se caractérise pratiquement la plupart du temps par une nécessité de croquer le médicament et de l'avaler ou encore de le broyer plus finement pour se l'injecter ou le priser.

Judicieusement la forme médicamenteuse préférée sera donc présentée sous forme divisée, le principe actif stupéfiant étant contenu dans un très grand nombre de microparticules de taille inférieure à 500 µm et de préférence inférieure à 300 µm. Sous cette forme, le broyage de petits objets sphériques est plus difficile que le broyage d'un simple comprimé matriciel dragéifié, et il devient pratiquement impossible de les croquer.

A titre d'exemples non limitatifs, on peut indiquer que la présente invention :
- s'applique aux PA appartenant à au moins l'une des familles de substances actives suivantes : amphétamines, analgésiques, anorexigènes, antalgiques, antidépresseurs, antiépileptiques, antimigraineux, antiparkinsoniens, antitussifs, anxiolytiques, barbituriques, benzodiazépines, hypnotiques, laxatifs, neuroleptiques, opiacés, psychostimulants, psychotropes, sédatifs, stimulants.
- s'applique aux composés choisis parmi les composés suivants: méthylphénidate, Fentanyl, Alfentanyl, Pentazocine, Péthidine, Phénopéridine, Rémifentanil, Sufentanil, Acétorphine, Acétylalphaméthylfentanyl, Acétylméthadol, Alfentanil, Allylprodine, Alphacétylméthadol, Alphaméprodine, Alphaméthadol, Alphaméthylfentanyl, Alpha-méthylthofentanyl, Alphaprodine, Aniléridine, atropine, Benzéthidine, Benzylmorphine, Bétahydroxyfentanyl, Béta-hydroxy-méthyl-3-fentanyl, Bétacétylméthadol, Bétaméprodine, Bétaméthadol, Bétaprodine, Bezitramide, buprénorphine, Butyrate de dioxaphétyl, Cannabis, Cétobémidone, Clonitazène, codéine, Coca, Cocaïne, Codoxime, Concentré de paille de pavot, Désomorphine, Dextromoramide, Diampromide, Diéthylthiambutène, Difénoxine, Dihydroétorphine, Dihydromorphine, Diménoxadol, Dimépheptanol, Diméthylthiambutène, Diphénoxylate, Dipipanone, Drotébanol, Ecgonine, éphédrine, Ethylméthylthiambutène, Etonitazène, Etorphine, Etoxéridine, Fentanyl, Furéthidine, Héroïne, Hydrocodone, Hydromorphinol, Hydromorphone, Hydroxypéthidine, Isométhadone, Lévométhorphane, Lévomoramide, Lévophénacylmorphane, Lévorphanol, meperidine, Métazocine, Méthadone, Méthyldésorphine, Méthyldihydromorphine, Méthyl-3-thiofentanyl, Méthyl-3-fentanyl, Métopon, Moramide, Morphéridine, morphine, MPPP, Myrophine, Nicomorphine, Noracyméthadol, Norlévorphanol, Norméthadone, Normorphine, Norpipanone, Opium, Oxycodone, Oxymorphone, Para-fluorofentanyl, PEPAP, Péthidine, Phénampromide, Phénazocine, Phénomorphane, Phénopéridine, Piminodine, Piritramide, Proheptazine, propanolol, Propéridine, Racéméthorphane, Racémoramide, Racémorphane, Rémifentanil, Sufentanil, Thébacone, Thébaïne, Thiofentanyl, Tilidine, Trimépéridine, Acétyldihydrocodéine, Codéine, Dextropropoxyphène, Dibydrocodéine, Ethylmorphine, Nicocodine, Nicodicodine, Norcodéine, Pholcodine, Propiram.
   et leurs mélanges.

### EXEMPLES

Les exemples suivants sont donnés à titre illustratif de la présente invention. Ils ne constituent en aucun cas une limite des possibilités.

### EXEMPLE 1 :

Dans cet exemple, c'est une forte viscosité de la solution résultant de la dissolution des excipients d'un médicament détourné qui est visée. 450g de microparticules de placébos, constituées de coeurs neutres de sucre, de diamètre compris entre 200 et 300 µm, sont pelliculés avec une solution S1 contenant 33,75g de polyvinylpyrrolidone (Kollidon 90F). 78,75g d'alginate de sodium et 1012,5g d'éthanol. 50g de ces microparticules sont ensuite mélangés à 50g d'une cire à bas point de fusion (Gelucire 44/14) 250mg de ces microparticules sont délayés dans 1mL d'eau ajustée à 0,1M CaCl₂ et pH neutre. La solution résultante est trop visqueuse pour être injectée. La forme broyée est pâteuse (non pulvérulente).

### EXEMPLE 2 :

50g des microparticules de l'exemple 1 sont mélangée à 50g d'une cire à bas point de fusion (Gelucire 44/14). On ajoute à cette préparation 50g de sphères de cellulose de diamètre compris entre 450 et 550 µm. Le broyage au mortier de cette préparation conduit à une pâte non pulvérulente. L'observation microscopique montre que les particules de sucre ont résisté au broyage.

### EXEMPLE 3 :

Dans cet exemple, une protection contre l'utilisation frauduleuse du médicament par voie nasale et injectable est visée. Pour cela, un mélange constitué de 100 mg de polyacide acrylique réticulé (Carbopol® 934P), de 160 mg de Diclofenac de sodium (à titre de principe actif modèle), de 100 mg de cire d'huile végétale hydrogénée (Lubritab®), de 10 mg de Stéarate de magnésium et 130 mg de lactose est comprimé.

Le broyage à sec de ces constituants mène à une pâte cireuse non pulvérulente empêchant son aspiration par voie nasale. La mise en solution de ce comprimé conduit à une solution trop visqueuse pour être injectée.

## Revendications

1. Forme médicamenteuse orale et solide, **caractérisée en ce qu'**elle comprend :
au moins un principe actif (PA) pharmaceutique, tout ou partie du principe actif de cette forme médicamenteuse étant contenu dans des microparticules,
des billes insolubles en milieu aqueux ou hydroalcoolique, incompressibles et inertes, de diamètre moyen supérieur ou égal à 1.25 fois, de préférence 1,5 fois, et plus préférentiellement encore 2 fois, le diamètre moyen des microparticules de principe actif,
ainsi que
A) au moins un agent mottant,
et
B) au moins un agent viscosifiant,
de manière à éviter le mésusage.

2. Forme médicamenteuse selon la revendication 1, **caractérisée en ce que** l'agent mottant A) est choisi parmi ceux aptes à faire en sorte, en cas de broyage de la forme médicamenteuse, que celle-ci se transforme en produit non-pulvérulent.

3. Forme médicamenteuse, selon la revendication 1 ou 2, **caractérisée en ce que** l'agent mottant A) est choisi dans la classe des composés hydrophobes agissant comme agent liant à sec.

4. Forme médicamenteuse selon l'une quelconque des revendications 1 à 3, **caractérisée en ce que** l'agent mottant A) est choisi dans la classe des composés hydrophobes agissant comme agent liant à sec:
→ dans le groupe comprenant les huiles de coton, les huiles de soja, les huiles de palme, les huiles de ricin et les mélanges de tout ou partie de ces huiles; et/ou
→ dans le groupe des cires, et plus préférentiellement encore dans le sous-groupe des cires comprenant les huiles de coton hydrogénées, les huiles de soja hydrogénées, les huiles de paume hydrogénées, les béhénates de glycérol, les huiles de ricin hydrogénées, les tristéarines, les tripalmitines, les trimyristines, les cires jaunes, les graisses dures, les matières grasses laitières anhydres, les lanolines, les palmitostéarates de glycérol, les stéarates de glycérol, les macrogolglycérides d'acide laurique, les alcools cétyliques, les diisostéarates de polyglycéryle, les monostéarates de diéthylène glycol, les monostéarates d'éthylène glycol, les omégas 3 et les mélanges de tout ou partie de ces cires; et/ou
→ dans le groupe des bases grasses pour suppositoires comprenant la glycérine, les triglycérides, les huiles de théobroma, les beurres de cacao et les mélanges de tout ou partie de ces produits.

5. Forme médicamenteuse selon l'une quelconque des revendications précédentes, **caractérisée en ce qu'**elle n'est pas transformable en une forme sèche administrable par aspiration nasale.

6. Forme médicamenteuse selon l'une quelconque des revendications précédentes, **caractérisée en ce que** l'agent viscosifiant B) est choisi parmi ceux aptes à rendre non injectable le PA contenu dans la forme médicamenteuse.

7. Forme médicamenteuse selon l'une quelconque des revendications précédentes, **caractérisée en ce que** l'agent viscosifiant B) est choisi dans les groupes de polymères suivants:
→ les polyacides acryliques et leurs dérivés, et/ou
→ les polyalkylènes glycols (e.g. polyéthylène glycol), et/ou
→ les polyvinylpyrrolidones, et/ou
→ les gélatines, et/ou
→ les polysaccharides, de préférence dans le sous-groupe comprenant l'alginate de sodium, les pectines, les guars, les xanthanes, les carraghénanes, les gellanes et les dérivés de la cellulose (e.g. hydroxypropylméthylcellulose, méthylcellulose, hydroxyéthylcellulose, carboxyméthylcellulose),
et leurs mélanges.

8. Forme médicamenteuse selon l'une quelconque des revendications précédentes, **caractérisée en ce qu'**elle n'est pas transformable en une forme injectable.

9. Forme médicamenteuse selon l'une quelconque des revendications précédentes **caractérisée en ce que** les billes insoluble inertes sont choisies dans le groupe de matériaux suivants: les celluloses et leurs dérivés insolubles, les résines polyméthacryliques et leurs dérivés, les silices, le talc, la semoule de blé, la bentonite et leurs mélanges.

10. Forme médicamenteuse selon l'une quelconque des revendications précédentes, **caractérisée en ce qu'**elle comprend du PA à libération immédiate et/ou du PA à libération modifiée.

11. Forme médicamenteuse selon la revendication 10,
**caractérisée en ce qu'**au moins une partie des microparticules sont des microcapsules à libération modifiée de PA.

12. Forme médicamenteuses selon l'une quelconque des revendications précédentes **caractérisée en ce que** les microparticules ou les microcapsules ont un diamètre moyen inférieur ou égal à 1000 µm, de préférence inférieur ou égal à 500 µm et plus préférentiellement de diamètre inférieur ou égal à 300 µm.

13. Forme médicamenteuse selon l'une quelconque des revendications précédentes, **caractérisée en ce que** l'extraction du PA par mastication et/ou broyage n'est pas efficace.

14. Forme médicamenteuse selon l'une quelconque des revendications précédentes **caractérisée en ce que** le PA mis en oeuvre appartient à au moins l'une des familles de substances actives suivantes : amphétamines, analgésiques, anorexigènes, antalgiques, antidépresseurs, antiépileptiques, antimigraineux, antiparkinsoniens, antitussifs, anxiolytiques, barbituriques, benzodiazépines, hypnotiques, laxatifs, neuroleptiques, opiacés, psychostimulants, psychotropes, sédatifs, stimulants.

15. Forme médicamenteuse selon l'une quelconque des revendications précédentes **caractérisée en ce que** le principe actif mis en oeuvre est choisi parmi les composés suivants:
Méthylphénidate, Pentazocine, Péthidine, Phénopéridine, Rémifentanil, Sufentanil, Acétorphine, Acétytalphaméthylfentanyl, Acétylméthadol, Alfentanil, Allylprodine, Alphacétylméthadol, Alphaméprodine, Alphaméthadol, Alphaméthylfentanyl, Alpha-méthylthiofentanyl, Alphaprodine, Aniléridine, Atropine, Benzéthidine, Benzylmorphine, Béta-hydroxyfentanyl, Béta-hydroxyméthyl-3-fentanyl, Bétacétylméthadol, Bétaméprodine, Bétaméthadol, Bétaprodine, Bezitramide, Buprénorphine, Butyrate de dioxaphétyl, Cannabis, Cétobémidone, Clonitazène, Codéine, Coca, Cocaïne, Codoxime, Concentré de paille de pavot. Désomorphine, Dextromoramide, Diampromide, Diéthylthiambutène, Difénoxine, Dihydroétorphine, Dihydromorphine, Diménoxadol, Dimépheptanol, Diméthylthiambutène, Diphénoxylate, Dipipanone, Drotébanol, Ecgonine, éphédrine, Ethylméthylthiambuténe, Etonitazène, Etorphine, Etoxéridine, Fentanyl, Furéthidine, Héroïne, Hydrocodone, Hydroinorphinol, Hydromorphone, Hydroxypéthidine, Isométhadone, Lévométhorphane, Lévomoramide, Lévophénacylmorphane, Lévorphanol, meperidine, Métazocine, Méthadone, Méthyldésorphine, Méthyldihydromorphine, Méthyl-3-thiofentanyl, Méthyl-3-fentanyl, Métopon, Moramide, Morphéridine, Morphine. MPPP, Myrophine, Nicomorphine, Noracyméthadol, Norlévorphanol, Norméthadone, Normorphine, Norpipanone, Opium, Oxycodone, Oxymorphone, Para-fluorofentanyl, PEPAP, Péthidine, Phénampromide, Phénazocine, Phénomorphane, Phénopéridine, Piminodine, Piritramide, Proheptazine, propanotol, Propéridine, Racéméthorphane, Racémoramide, Racémorphane, Rémifentanil, Sufentanil, Thébacone, Thébaïne, Thiofentanyl, Tilidine, Trimépéridine, Acétyldihydrocodéine, Dextropropoxyphène, Dihydrocodéine, Ethylmorphine, Nicocodine, Nicodicodine, Norcodéine, Pholcodine, Propiram, et leurs mélanges.

## Claims

1. Solid oral drug form, **characterized in that** it comprises:
at least one pharmaceutical active ingredient AI, all or part of the active ingredient of this drug form being contained in microparticles,
beads insoluble in an aqueous or aqueous-alcoholic medium, which cannot be compressed and are inert, which have an average diameter of greater than or equal to 1.25 times, preferably 1.5 times, and even more preferably twice, the average diameter of the microparticles of active ingredient,
and also
A) at least one caking agent,
and
B) at least one viscosifying agent;
so as to prevent misuse.

2. Drug form according to claim 1, **characterized in that** the caking agent A) is chosen from those capable of ensuring, in the event of crushing of the drug form, that the latter is converted into a non-pulverulent product.

3. Drug form according to claim 1 or 2, **characterized in that** the caking agent A) is chosen from the class of hydrophobic compounds that act as a dry binder.

4. Drug form according to any one of claims 1 to 3, **characterized in that** the caking agent A) is chosen from the class of hydrophobic compounds that act as a dry binder, preferably:
→ from the group comprising cottonseed oils, soybean oils, palm oils, castor oils and mixtures of all or some of these oils; and/or
→ from the group of waxes, and even more preferably from the subgroup of waxes comprising hydrogenated cottonseed oils, hydrogenated soybean oils, hydrogenated palm oils, glyceryl behenates, hydrogenated castor oils, tristearins, tripalmitins, trimyristins, yellow waxes, hard fats, anhydrous dairy fats, lanolins, glyceryl palmitostearates, glyceryl stearates, lauric acid macrogolglycerides, cetyl alcohols, polyglyceryl diisostearates, diethylene glycol monostearates, ethylene glycol monostearates, omegas 3 and mixtures of all or some of these waxes; and/or
→ from the group of fatty bases for suppositories comprising glycerol, triglycerides, theobroma oils, cacao butters and mixtures of all or some of these products.

5. Drug form according to any one of the preceding claims, **characterized in that** it cannot be converted into a dry form that can be administered by nasal aspiration.

6. Drug form according to any one of the preceding claims, **characterized in that** the viscosifying agent B) is chosen from those capable of rendering non-injectable the AI contained in the drug form.

7. Drug form according to any one of the preceding claims, **characterized in that** the viscosifying agent B) is chosen from the following groups of polymers:
→ polyacrylic acids and derivatives thereof, and/or
→ polyalkylene glycols (e.g. polyethylene glycol), and/or
→ polyvinylpyrrolidones, and/or
→ gelatins, and/or
→ polysaccharides, preferably from the subgroup comprising: sodium alginate, pectins, guars, xanthans, carrageenans, gellans and cellulose derivatives (e.g. hydroxypropylmethylcellulose, methylcellulose, hydroxyethylcellulose, carboxymethylcellulose),
and mixtures thereof.

8. Drug form according to any one of the preceding claims, **characterized in that** it cannot be converted into an injectable form.

9. Drug form according to any one of the preceding claims, **characterized in that** the insoluble inert beads are chosen from the group of following substances:
celluloses and insoluble derivatives thereof, polymethacrylic resins and derivatives thereof, silicas, talc, semolina, bentonite and mixtures thereof.

10. Drug form according to any one of the preceding claims, **characterized in that** it comprises immediate-release AI and/or modified-release AI.

11. Drug form according to claim 10, **characterized in that** at least some of the microparticles are microcapsules for modified release of AI.

12. Drug form according to any one of the preceding claims, **characterized in that** the microparticles or the microcapsules have an average diameter of less than or equal to 1000 µm, preferably less than or equal to 500 µm, and more preferably a diameter of less than or equal to 300 µm.

13. Drug form according to any one of the preceding claims, **characterized in that** the extraction of the AI by chewing and/or crushing is not effective.

14. Drug form according to any one of the preceding claims, **characterized in that** the AI used belongs to at least one of the following families of active substances: amphetamines, analgesics, appetite suppressants, antalgics, antidepressants, antiepileptics, antimigraine agents, antiparkinsonian agents, antitussives, anxiolytics, barbiturates, benzodiazepines, hypnotics, laxatives, neuroleptics, opiates, psychostimulants, psychotropic agents, sedatives and stimulants.

15. Drug form according to any one of the preceding claims, **characterized in that** the active ingredient used is chosen from the following compounds:
Methylphenidate, Pentazocine, Pethidine, Phenoperidine, Remifentanil, Sufentanil, Acetorphine, Acetylalphamethylfentanyl, Acetylmethadol, Alfentanil, Allylprodine, Alphacetylmethadol, Alphameprodine, Alphamethadol, Alphamethylfentanyl, Alpha-methylthiofentanyl, Alphaprodine, Anileridine, Atropine, Benzethidine, Benzylmorphine, Beta-hydroxyfentanyl, Beta-hydroxymethyl-3-fentanyl, Betacetylmethadol, Betameprodine, Betamethadol, Betaprodine, Bezitramide, buprenorphine, Dioxaphentyl butyrate, Cannabis, Ketobemidone, Clonitazene, Codeine, Coca, Cocaine, Codoxime, Concentrate of poppy straw, Desomorphine, Dextromoramide, Diampromide, Diethylthiambutene, Difenoxine, Dihydroetorphine, Dihydromorphine, Dimenoxadol, Dimepheptanol, Dimethylthiambutene, Diphenoxylate, Dipipanone, Drotebanol, Ecgonine, ephedrine, Ethylmethylthiambutene, Etonitazene, Etorphine, Etoxeridine, Furethidine, Heroin, Hydrocodone, Hydromorphinol, Hydromorphone, Hydroxypethidine, Isomethadone, Levomethorphane, Levomoramide, Levophenacylmorphane, Levorphanol, meperidine, Metazocine, Methadone, Methyldesorphine, Methyldihydromorphine, Methyl-3-thiofentanyl, Methyl-3-fentanyl, Metopon, Moramide, Morpheridine, morphine, MPPP, Myrophine, Nicomorphine, Noracymethadol, Norlevorphanol, Normethadone, Normorphine, Norpipanone, Opium, Oxycodone, Oxymorphone, Para-fluorofentanyl, PEPAP, Pethidine, Phenampromide, Phenazocine, Phenomorphane, Phenoperidine, Piminodine, Piritramide, Proheptazine, propanolol, Properidine, Racemethorphane, Racemoramide, Racemorphane, Remifentanil, Sufentanil, Thebacone, Thebaine, Thiofentanyl, Tilidine, Trimeperidine, Acetyldihydrocodeine, Dextropropoxyphene, Dihydrocodeine, Ethylmorphine, Nicocodine, Nicodicodine, Norcodeine, Pholcodine, Propiram, and mixtures thereof.

## Patentansprüche

1. Orale und feste Arzneimittelform, **dadurch gekennzeichnet, dass** sie Folgendes umfasst:
mindestens einen pharmazeutischen Wirkstoff (WS), wobei der gesamte oder ein Teil des Wirkstoffs dieser Arzneimittelform in Mikropartikeln enthalten ist,
Kügelchen, die inkompressibel, inert und in wässrigem oder hydroalkoholischem Medium unlöslich, sind, mit einem mittleren Durchmesser von mehr als oder gleich dem 1,25-Fachen, vorzugsweise dem 1,5-Fachen und ganz besonders bevorzugt dem 2-Fachen des mittleren Durchmessers der Mikropartikeln des Wirkstoffs sowie A) mindestens ein Antiklumpmittel
und
B) mindestens ein viskositätserhöhendes Mittel,
um Missbrauch zu verhindern.

2. Arzneimittelform nach Anspruch 1, **dadurch gekennzeichnet, dass** das Antiklumpmittel A) aus denjenigen ausgewählt ist, die im Falle einer Zerkleinerung der Arzneimittelform sicherstellen können, dass diese sich in ein nicht-pulverförmiges Produkt umwandelt.

3. Arzneimittelform, nach Anspruch 1 oder 2, **dadurch gekennzeichnet, dass** das Antiklumpmittel A) aus der Klasse der hydrophoben Verbindungen ausgewählt ist, die als Trockenbindemittel wirken.

4. Arzneimittelform nach einem der Ansprüche 1 bis 3, **dadurch gekennzeichnet, dass** das Antiklumpmittel A) aus der Klasse der hydrophoben Verbindungen ausgewählt ist, die als Trockenbindemittel wirken:
- aus der Gruppe, die Baumwollsamenöle, Sojaöle, Palmöle, Ricinusöle und die Gemische von allen oder einem Teil dieser Öle umfasst; und/oder
- aus der Gruppe der Wachse und ganz besonders bevorzugt aus der Untergruppe der Wachse, die hydrierte Baumwollsamenöle, hydrierte Sojaöle, hydrierte Palmöle, Glycerinbehenate, hydrierte Ricinusöle, Tristearine, Tripalmitine, Trimyristine, gelbe Wachse, Hartfette, wasserfreie Milchfette, Lanoline, Glycerinpalmitostearate, Glycerinstearate, Laurinsäuremacrogolglyceride, Cetyl-alkohole, Polyglyceryldiisostearate, Diethylenglycolmonostearate, Ethylenglycolmonostearate, Omega 3 und die Gemische von allen oder einem Teil dieser Wachse umfasst; und/oder
- aus der Gruppe der Fettbasen für Suppositorien, die Glycerin, Triglyceride, Theobromaöle, Kakaobutter und die Gemische von allen oder einem Teil dieser Produkte umfasst.

5. Arzneimittelform nach einem der vorhergehenden Ansprüche, **dadurch gekennzeichnet, dass** sie nicht in eine trockene Form überführbar ist, die durch Einatmen durch die Nase verabreicht werden kann.

6. Arzneimittelform nach einem der vorhergehenden Ansprüche, **dadurch gekennzeichnet, dass** das viskositätserhöhende Mittel B) aus denjenigen ausgewählt ist, die den in der Arzneimittelform enthaltenen WS nichtinjizierbar machen können.

7. Arzneimittelform nach einem der vorhergehenden Ansprüche, **dadurch gekennzeichnet, dass** das viskositätserhöhende Mittel B) aus den folgenden Gruppen von Polymeren ausgewählt ist:
- Polyacrylsäuren und ihren Derivate und/oder
- Polyalkylenglycolen (z. B. Polyethylenglycol) und/oder
- Polyvinylpyrrolidonen und/oder
- Gelatinen und/oder
- Polysacchariden, vorzugsweise aus der Untergruppe, die Folgende umfasst: Natriumalginat, Pektine, Guarkernmehle, Xanthane, Carrageenane, Gellane und Cellulosederivate (z. B. Hydroxypropylmethylcellulose, Methylcellulose, Hydroxyethylcellulose, Carboxymethylcellulose),
und ihren Gemischen.

8. Arzneimittelform nach einem der vorhergehenden Ansprüche, **dadurch gekennzeichnet, dass** sie nicht in eine injizierbare Form überführbar ist.

9. Arzneimittelform nach einem der vorhergehenden Ansprüche, **dadurch gekennzeichnet, dass** die unlöslichen inerten Kügelchen aus der Gruppe der folgenden Materialien ausgewählt sind: Cellulosen und ihren unlöslichen Derivaten, Polymethacrylharzen und ihren Derivaten, Kieselsäuren, Talk, Weizengries, Bentonit und ihren Gemischen.

10. Arzneimittelform nach einem der vorhergehenden Ansprüche, **dadurch gekennzeichnet, dass** sie WS mit sofortiger Freisetzung und/oder WS mit modifizierter Freisetzung umfasst.

11. Arzneimittelform nach Anspruch 10, **dadurch gekennzeichnet, dass** es sich bei mindestens einem Teil der Mikropartikel um Mikrokapseln mit modifizierter Freisetzung des WS handelt.

12. Arzneimittelform nach nach einem der vorhergehenden Ansprüche, **dadurch gekennzeichnet, dass** die Mikropartikeln oder die Mikrokapseln einen mittleren Durchmesser haben, der kleiner oder gleich 1000 µm, vorzugsweise kleiner oder gleich 500 µm ist, und noch stärker bevorzugt einen Durchmesser, der kleiner oder gleich 300 µm ist.

13. Arzneimittelform nach einem der vorhergehenden Ansprüche, **dadurch gekennzeichnet, dass** die Extraktion des WS durch Zerkauen und/oder Zerkleinern nicht wirksam ist.

14. Arzneimittelform nach einem der vorhergehenden Ansprüche, **dadurch gekennzeichnet, dass** der verwendete WS zu mindestens einer der folgenden Familien von Wirksubstanzen gehört: Amphetamine, Analgetika, Anorexigene, Schmerzmittel, Antidepressiva, Antiepileptika, Antimigränemittel, Antiparkinsonmittel, Antitussiva, Anxiolytika, Barbiturate, Benzodiazepine, Hypnotika, Laxative, Neuroleptika, Opiate, Psychostimulantien, psychotrope Mittel, Sedativa, Stimulantien.

15. Arzneimittelform nach einem der vorhergehenden Ansprüche, **dadurch gekennzeichnet, dass** der verwendete Wirkstoff aus den folgenden Substanzen ausgewählt ist:
Methylphenidat, Pentazocin, Pethidin, Phenoperidin, Remifentanil, Sufentanil, Acetorphin, Acetylalphamethylfentanyl, Acetylmethadol, Alfentanil, Allylprodin, Alphacetylmethadol, Alphameprodin, Alphamethadol, Alphamethylfentanyl, Alpha-Methylthiofentanyl, Alphaprodin, Anileridin, Atropin, Benzethidin, Benzylmorphin, Beta-Hydroxyfentanyl, Beta-Hydroxymethyl-3-fentanyl, Betacetylmethadol, Betameprodin, Betamethadol, Betaprodin, Bezitramid, Buprenorphin, Dioxaphetylbutyrat, Cannabis, Ketobemidon, Clonitazen, Codein, Koka, Kokain, Codoxim, Mohnstrohkonzentrat, Desomorphin, Dextromoramid, Diampromid, Diethylthiambuten, Difenoxin, Dihydroetorphin, Dihydromorphin, Dimenoxadol, Dimepheptanol, Dimethylthiambuten, Diphenoxylat, Dipipanon, Drotebanol, Ecgonin, Ephedrin, Ethylmethylthiambuten, Etonitazen, Etorphin, Etoxeridin, Fentanyl, Furethidin, Heroin, Hydrocodon, Hydromorphinol, Hydromorphon, Hydroxypethidin, Isomethadon, Levomethorphan, Levomoramid, Levophenacylmorphan, Levorphanol, Meperidin, Metazocin, Methadon, Methyldesorphin, Methyldihydromorphin, Methyl-3-thiofentanyl, Methyl-3-fentanyl, Metopon, Moramid, Morpheridin, Morphin, MPPP, Myrophin, Nicomorphin, Noracymethadol, Norlevorphanol, Normethadon, Normorphin, Norpipanon, Opium, Oxycodon, Oxymorphon, Parafluorfentanyl, PEPAP, Pethidin, Phenampromid, Phenazocin, Phenomorphan, Phenoperidin, Piminodin, Piritramid, Proheptazin, Propanolol, Properidin, Racemethorphan, Racemoramid, Racemorphan, Remifentanil, Sufentanil, Thebacon, Thebain, Thiofentanyl, Tilidin, Trimeperidin, Acetyldihydrocodein, Dextropropoxyphen, Dihydrocodein, Ethylmorphin, Nicocodin, Nicodicodin, Norcodein, Pholcodin, Propiram
und ihren Gemischen.
